# EUROPEAN PATENT SPECIFICATION

(11) **EP 2 533 870 B1**
(45) Date of publication and mention of the grant of the patent: **02.08.2017**
(21) Application number: 11700565.2
(22) Date of filing: 21.01.2011
(51) Int. Cl.: B01D 1/06, B01D 1/14, B01D 3/04, C07C 273/04, B01D 3/00

(54) **Falling-film stripper and stripping medium distributor for carbamate decomposition**
Fallfilmabstreifer für Carbamatzersetzung
Nettoyeur à film ruisselant pour la décomposition du carbamate

(30) Priority: 12.02.2010 EP 10153498
(43) Date of publication of application: 19.12.2012
(73) Proprietor: CASALE SA, 6900 Lugano (CH)
(72) Inventor: SCOTTO, Andrea, CH-6932 Breganzona (CH); VISCIOTTI, Damiano, I-20146 Milano (IT)
(74) Representative: Zardi, Marco
(86) International application number: PCT/EP2011/050796
(87) International publication number: WO 2011/098335

(56) References cited:
- EP-A1- 1 195 194
- EP-A1- 1 516 664
- US-A- 4 788 020

## Description

### Field of the invention

The invention relates to a falling film stripper for carbamate decomposition and ammonia plus carbon dioxide recovery from the effluent of a reactor for synthesis of urea. More in detail, the invention relates to distribution of a gaseous stripping medium, such as carbon dioxide, in the tubes of the stripper.

### Prior Art

In a process for the production of urea, it is known to feed the reactor effluent to a stripper, where carbamate is decomposed to ammonia and carbon dioxide with the help of heat and of a stripping agent.

A falling-film stripper is basically a vertical shell-and-tube heat exchanger, comprising a top chamber and a bottom chamber, in fluid communication by means of the tubes of a tube bundle. The upper chamber is fed with the reactor effluent; the lower chamber is fed with the stripping medium and collects the concentrated urea solution from the tubes. A gaseous phase comprising the stripping medium and the ammonia vapors (overhead vapors) is collected in the upper part of the top chamber. The top ends of the tubes are usually fitted with ferrules and suitable openings to allow the inlet of the liquid and the outlet of the gaseous phase. In operation, the liquid urea solution forms a falling film on the inner surface of tubes, while the gaseous stripping medium flows in counter-current from bottom to top, in the same tubes.

The tubes are heated in operation by a suitable heating medium, e.g. by condensing steam. The stripping medium is for example carbon dioxide.

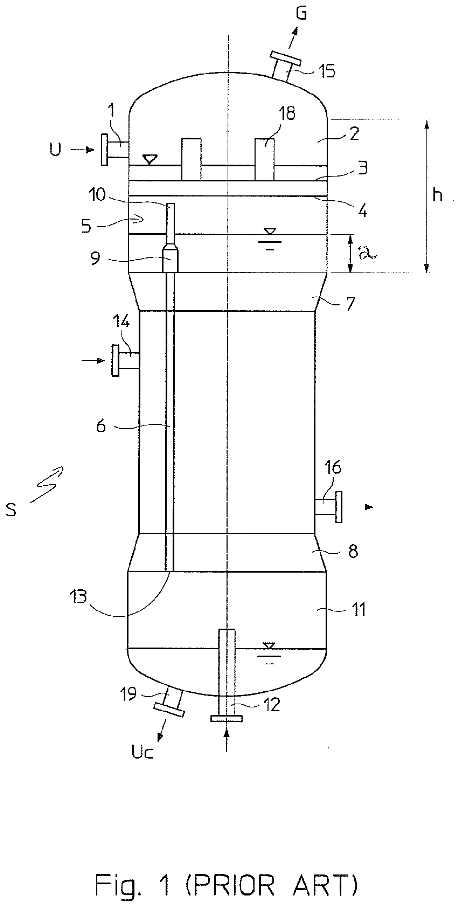

A specific example of a prior art CO₂ stripper S is shown in Fig. 1. The urea solution U (reactor effluent) enters via the top inlet 1 in a top chamber 2, and is poured through a perforated tray 3 and an intermediate grid 4, over the tubes 6 of a steam-heated tube bundle. The tubes 6 of the bundle are fixed at opposite ends to an upper and lower tube sheet 7, 8. The tube bundle may have hundreds or thousands of tubes, only one tube 6 is shown for simplicity.

The top ends of tubes 6 are fitted with ferrules 5. The urea solution U enters the tubes 6 via suitable liquid inlets holes in the bottom part 9 of said ferrules 5, forming a liquid thin film on the internal surface of each tube. The opposite ends of tubes 6 are open in a bottom chamber 11, in communication with a CO₂ inlet 12. The carbon dioxide enters the tubes 6 at the bottom open ends 13, flowing in counter-current with the liquid film. The tubes 6 are heated by condensing steam fed to the shell side of the stripper S, entering at a steam inlet 14 and exiting at a condensate outlet 16.

A concentrated urea solution U_{C} is collected in the chamber 11 and directed to the liquid outlet 19. A gaseous phase G containing ammonia and carbon dioxide flows from the top ends of tubes 6 and gas distributors 18, to an outlet 15, and is usually directed to a condenser or to a reactor.

The distribution of the CO₂ feed throughout the tubes of the tube bundle is mainly governed by the concentrated pressure drop occurring at the outlet of the gaseous phase, e.g. in Fig. 1 at the gas outlets of the upper portion 10 of the ferrules 5. These gas outlets are designed to provide a given pressure drop, suitable to ensure proper distribution of the carbon dioxide feed in all the tubes.

In operation, the stripper needs a certain liquid head ***a*** over the tube sheet 7, in order to support the downwards liquid flow. This liquid head depends on both the pressure drop of the liquid phase, and the pressure drop of the gaseous phase.

The above details are well known to a skilled person and therefore will not be further discussed. This design of a falling-film stripper has however some disadvantages that have not been solved yet.

It has been noted that the liquid film, while flowing out from the bottom ends of tubes, tends to "close" or reduce the section of the tubes available for the feed of the stripping medium. The distribution of the stripping medium throughout the tubes of the tube bundles may suffer a lack of uniformity, causing a loss of efficiency of the overall stripping process. Further, it has been noted that the stripping medium entering the bottom of tubes causes some disturbance to the counter-current outlet of the liquid phase and in some cases a back-up liquid flow has been observed, also in this case with loss of efficiency.

A further drawback is that the upper part of the stripper must be designed to accommodate ferrules of a sufficient length, in order to allow the outlet of the gaseous phase in the presence of the necessary liquid level in the top chamber. Referring to Fig. 1, the ferrules 5 must have a sufficient length to keep the gas outlets of the upper portions 10 over the liquid level. Typically the required liquid head ***a*** is around 400 to 800 mm, typically 500 mm, and consequently the ferrules are almost 1 m long. Hence, the whole upper part of the high-pressure stripper is higher, i.e. the overall dimension ***h*** is influenced by the need of the liquid head ***a*** over the tubes. This is a disadvantage especially because the stripper operates under high pressure and needs expensive materials to avoid corrosion, hence the manufacturing of large parts is expensive.

It should also be considered that a falling-film stripper is sensitive to the so-called flooding. Flooding occurs when the liquid film inside the tubes is broken down and part of the liquid is entrained by the exiting gas stream. Flooding must be avoided as it reduces the stripping efficiency. This problem is encountered e.g. when a urea plant is revamped to increase the production rate. It may happen that the existing stripper cannot support the increased flow rate, due to the flooding.

An apparatus for effecting mass transfer between liquid and gas phases is disclosed in US-A-4 788 020 while an apparatus for carbamate decomposition and NH3 and CO2 stripping from urea solutions is disclosed in EP-A-1 195 194.

### Summary of the invention

The aim of the invention is to solve the above drawbacks of falling-film strippers for carbamate decomposition, by improving the distribution of the stripping medium into the tubes.

The aim is achieved by distributing the stripping medium with a plurality of gas risers located in the bottom part of the stripper. Each gas riser feeds a respective tube of the tube bundle with the stripping medium. The gas risers are tubes smaller than the tubes of the main tube bundle, with an end entering into each tube of the stripper, to provide a direct feeding of the stripping medium to said tube.

Accordingly, a shell-and-tube stripper for carbamate decomposition and ammonia recovery from a urea solution, comprising a top chamber in communication with an inlet for said urea solution; a bundle of heated tubes having top ends in communication with the top chamber, and bottom ends in communication with a feed of a gaseous stripping medium, and where in operation a liquid falling film of said urea solution and a counter-current gaseous flow of the stripping medium are established inside the tubes, is characterized in that said feed of the stripping medium is in communication with bottom sections of the tubes by means of a plurality of gas risers disposed to distribute the stripping medium in respective tubes, each gas riser being realized with a tube smaller than said tubes of the tube bundle, having a first end in communication with the feed of the stripping medium, and a second end into a respective tube of the tube bundle.

Preferably, the stripper comprises a gas riser for each of the tubes of the tube bundle. Preferably, the gas risers are coaxial with the tubes of the tube bundle.

The gas risers are smaller than the stripping tubes, so that an annular space for liquid outlet is left around the gas riser, at the bottom end of each stripping tube. The outer diameter of the gas risers hence is less than the inner diameter of the stripping tubes.

In a preferred embodiment, the stripper has a bottom chamber which is in communication with the feed of the stripping medium, and collects the concentrated urea solution flowing down from the tubes; the gas risers are preferably associated to a tray located in said bottom chamber below the bottom ends of the tubes. In some embodiments of the invention, said tray is perforated to drain the concentrated urea solution, so that the solution is collected beneath the tray itself. In alternative embodiments, the concentrated urea solution is drained from said tray by overflowing from a downcomer associated to the tray.

The stripping medium is preferably carbon dioxide.

The stripper according to the invention is generally part of a high-pressure loop comprising a reactor, a stripping section and optionally a condensation section. Hence, an aspect of the invention is a plant for the synthesis of urea, where the high-pressure loop comprises the above disclosed stripper.

A further aspect of the invention is a method for carbamate decomposition and ammonia recovery from a urea solution, where said solution is fed to tubes of a falling-film tube bundle vertical stripper, in counter-current with a gaseous stripping medium; a concentrated urea solution is collected at the bottom of said stripper, and a gaseous phase containing the carbon dioxide and ammonia is recovered at the top of said stripper, the method being characterized in that the stripping medium is fed to said tubes by means of a plurality gas risers, each gas riser being realized with a tube smaller than said tubes of the tube bundle, each gas riser having one end in communication with a feed of the stripping medium, and a second end into a respective tube of the tube bundle.

A further aspect of the invention is the revamping of an existing stripper, in particular the high-pressure CO₂ stripper in a CO₂-stripping urea process. An aspect of the invention is the revamping of an existing stripper by the provision of at least the aforesaid gas risers, each gas riser being disposed to enter into a respective tube of the tube bundle of the stripper, for achieving a direct feed of the stripping medium into the tubes. Preferably, the gas risers are provided in association with a perforated tray which is placed in a bottom chamber of the stripper, connected to the carbon dioxide feed and where the concentrated urea solution is collected.

An advantage of the invention is the uniform distribution of the gaseous stripping medium in the tubes of the tube bundle. The gas risers provide direct feed of the stripping medium to the respective tubes, and injection of the stripping medium beyond the liquid outlet section, at the bottom end of the tubes. Hence, the interference between the liquid outlet and the gas inlet, at the bottom end of the tubes, is eliminated or significantly reduced. The flow rate of the gaseous stripping medium is substantially the same in all tubes, and hence the process is more efficient. Uniform distribution of the stripping medium reduces the risk of flooding and allows a full exploitation of the available heat exchange surface. In other words, the stripper is made more efficient in relation to number and dimension of tubes.

A further and notable advantage is that the gas risers provide the concentrated pressure drop governing the distribution of the stripping medium. This pressure drop, according to the invention, is located in the lower part of the stripper, namely at the inlet of the stripping medium into the tubes, contrary to the prior art where the main pressure drop is in the upper part of the stripper, at the gas outlet. This means that the ferrules at the top of the tube bundle can be made shorter, with larger gas outlets than in the prior art; the overall design of the upper part of the stripper can be made more compact and less expensive.

The invention and the related advantages will be more evident with the help of the following description of a preferred embodiment.

### Brief description of the drawings

Fig. 1 is scheme of a prior art stripper.
Fig. 2 is a scheme of a shell-and-tube, steam-heated stripper according to an embodiment of the invention.
Fig. 3 is an enlarged view of the detail III in Fig. 2, showing the upper end of one gas riser entering in a respective tube of the tube bundle.
Fig. 4 is a cross section according to line IV-IV of Fig. 3.
Fig. 5 is a detail of a gas riser entering a respective tube according to another embodiment of the invention.

### Detailed description

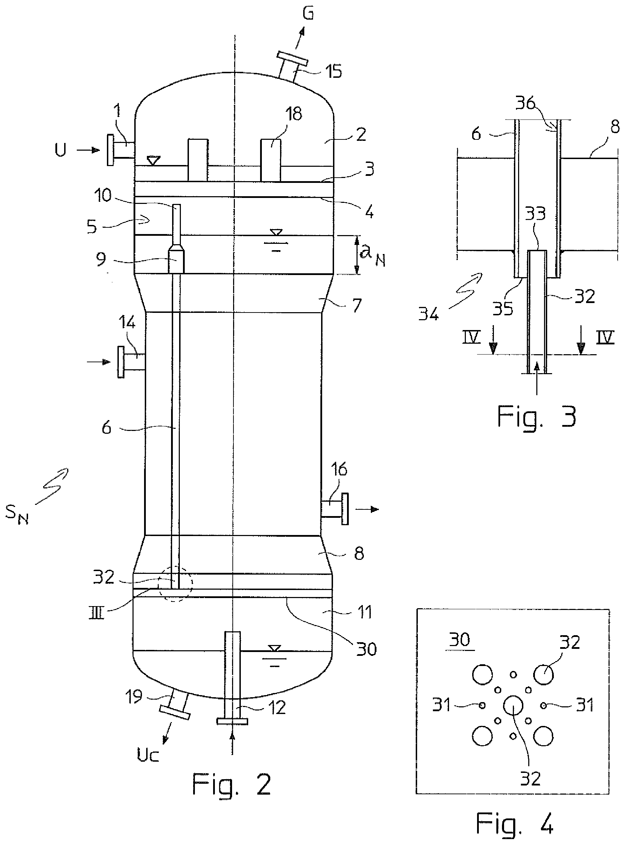

A stripper S_{N} according to the invention is shown in Fig. 2. The features equivalent to those of the known stripper of Fig. 1 are indicated with the same numerals and only the new features will be now discussed.

In the bottom liquid chamber 11, the inventive stripper S_{N} has a perforated tray 30 with liquid drain holes 31 (Fig. 4) and carrying a plurality of gas risers 32. Each gas riser 32 is a tube smaller than tubes 6 of the main tube bundle, with a bottom end associated to the tray 30 and in communication with the chamber 11, and a top end section 33 entering into a respective tube 6, over the bottom end section 34 of said tube 6 (Fig. 3).

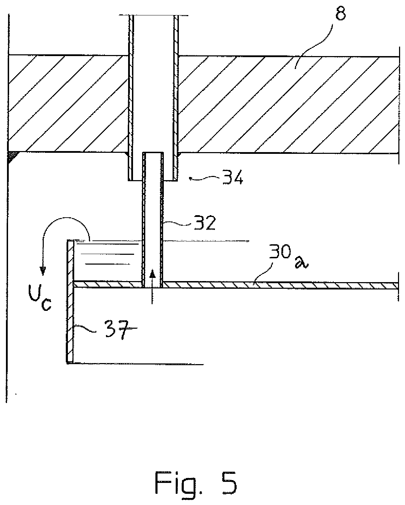

According to the embodiment of Fig. 5, the gas risers 32 are supported by a tray 30a without liquid drain holes. The concentrated urea solution Uc accumulates on the tray 30a and overflows from a downcomer 37.

An annular space 35 is left around the gas riser 32, at said bottom end section 34 of the tube 6. For example, the inner diameter of tubes 6 is 25 mm, while the outer diameter of gas risers 32 is 15 mm. Said annular space 35 allows the liquid film, which in operation is formed on the internal surface 36 of tube 6, to flow down in the chamber 11. The concentrated urea solution Uc in the bottom chamber 11 is discharged through the outlet 19 and directed to further treatment, e.g. to a downstream section at lower pressure.

It should be noted that the pressure drop of the stripping medium is substantially moved from the top of the stripper, i.e. the outlets on the ferrule portions 10, to the bottom, i.e. the passage through the gas risers 32. As a consequence, the liquid head over the tubes 6 must overcome pressure drop of the gas stream significantly lower through the ferrules 5; in other words, the novel stripper S_{N} requires a liquid head ***a_{N}*** which is substantially less than the liquid head ***a*** of an equivalent prior-art stripper. For example, the head ***a_{N}*** can be around 50% of the prior-art head ***a***.

Accordingly, the design of the stripper can be advantageously modified: the gas outlet opening on the upper portions 10 of the ferrules 5 can be larger, as a less pressure drop is required; the ferrules 5 can be shorter and the overall dimension ***h*** (Fig. 1) can be reduced. In practice, said dimension ***h*** may be about 500 mm shorter. A new stripper can be made more compact than in the prior art, this being a notable advantage because the stripper of a urea plant is a high-pressure and expensive item.

If the stripper S_{N} is obtained after a revamping of an existing unit, it may be preferable to maintain the existing vessel. The invention is still advantageous, because the modified stripper is less sensitive to flooding, thanks to the uniform distribution of the stripping medium in the tubes and the elimination of the interference between the liquid outlet and gas inlet at the bottom of tubes 6. Hence, the available heat exchange surface is used in an optimal manner and the capacity of the stripper is still increased, at a low cost and without further modifications.

The stripper S_{N} operates as follows. The reactor effluent U, which is an aqueous solution of urea, carbamate and unconverted free ammonia, enters the tubes 6 via tangential liquid inlet holes on the lower parts 9 of the ferrules 5, thus forming a liquid film on the walls 36 of tubes 6. The stripping medium, such as carbon dioxide, is fed via inlet duct 12 and gas risers 32, to the bottom of the same tubes 6, flowing in counter-current with the liquid film. Under the action of the stripping medium and surface heating, the carbamate is dissociated and unreacted ammonia and carbon dioxide are "stripped" from the solution and recovered as the overhead vapors G. The concentrated urea solution Uc is obtained at the bottom end of the stripper.

## Claims

1. A plant for the synthesis of urea, comprising at least a reactor and a stripping section in a high-pressure loop, the stripping section comprising at least one shell-and-tube stripper (S_{N}) for carbamate decomposition and ammonia recovery from a urea solution (U), the stripper comprising a top chamber (2) in communication with an inlet (1) for said urea solution; a bundle of heated tubes (6), said tubes (6) having top ends in communication with the top chamber (2), and bottom ends (13) in communication with a feed (12) of a gaseous stripping medium, where in operation a liquid falling film of said urea solution and a counter-current gaseous flow of the stripping medium are established inside the tubes (6), **characterized in that** said feed (12) of the stripping medium is in communication with bottom end sections (34) of the tubes (6) by means of a plurality of gas risers (32) disposed to distribute the stripping medium in respective tubes, each gas riser being realized with a tube smaller than said tubes of the tube bundle, having a first end in communication with the feed (12) of the stripping medium, and a second end (33) into a respective tube (6) of the tube bundle; and wherein ferrules (5) are fitted at the top ends of said tubes (6).

2. A plant according to claim 1, comprising a gas riser (32) for each of the tubes (6) of said tube bundle.

3. A plant according to claim 1 or 2, the gas risers (32) being coaxial with the tubes (6) of said tube bundle.

4. A plant according to any of claims 1 to 3, the gas risers (32) being associated to a tray (30, 30a) in a bottom chamber (11) of the stripper, said bottom chamber (11) being in communication with the feed (12) of the stripping medium.

5. A plant according to claim 4, said tray being a perforated tray (30) or being associated to a downcomer (37), to drain a liquid phase of concentrated urea solution (Uc) falling from the tubes (6) from said tray to the bottom chamber (11) below.

6. A method for revamping a CO₂ stripper of a plant for the synthesis of urea, said stripper to be revamped comprising an inlet of a urea solution coming from a synthesis reactor, a carbon dioxide feed, a vertical tube bundle of surface-heated tubes where the urea solution is fed in counter-current with the carbon dioxide and ferrules fitted at the top of the tube bundles, the method **characterised in** the the provision of a plurality of gas risers (32) disposed to distribute the carbon dioxide in respective tubes of the bundle, the gas risers being tubes smaller than the heated tubes, each gas riser having one end in communication with said carbon dioxide feed, and a second end extending into a respective tube of the tube bundle.

7. A method according to claim 6, the gas risers being provided in association with a tray (30, 30a), said tray being mounted in a bottom chamber (11) of the stripper, for collecting the concentrated urea solution and in communication with the carbon dioxide feed.

8. A method according to claim 6, each tube of the tube bundle being directly fed with the stripping medium by means of a respective gas riser (32).

## Patentansprüche

1. Anlage für die Harnstoffsynthese, umfassend mindestens einen Reaktor und einen Abstreiferabschnitt in einem Hochdruck-Kreislauf, wobei der Abstreiferabschnitt mindestens einen Röhrenabstreifer (S_{N}) zum Carbamatabbau und Ammoniak-Rückgewinnung aus einer Harnstofflösung (U) umfasst, wobei der Abstreifer eine obere Kammer (2) aufweist, die mit einem Einlass (1) für die Harnstofflösung in Verbindung steht; ein Bündel von beheizten Röhren (6), wobei obere Enden der Röhren (6) mit der oberen Kammer (2) in Verbindung stehen, und untere Enden (13) mit einer Zuführung (12) eines gasförmigen Abstreifmediums in Verbindung stehen, wobei in Betrieb ein flüssiger Fallfilm der Harnstofflösung und ein gasförmiger Gegenstrom des Abstreifmediums in dem Inneren der Röhren (6) aufgebaut sind, **dadurch gekennzeichnet, dass** die Zuführung (12) des Abstreifmediums mit den unteren Endabschnitten (34) der Röhren (6) mittels einer Vielzahl von Gassteigrohren (32) in Verbindung steht, die angeordnet sind, um das Abstreifmedium in jeweiligen Röhren zu verteilen, wobei jedes Gassteigrohr mit einer solchen Röhre ausgebildet ist, die kleiner als die Röhren des Röhrenbündels ist und ein erstes Ende in Verbindung mit der Zuführung (12) des Abstreifmediums und ein zweites Ende (33) in eine jeweilige Röhre (6) des Röhrenbündels aufweist; und wobei Hülsen (5) auf den oberen Enden der Röhren (6) montiert sind.

2. Anlage nach Anspruch 1, umfassend ein Gassteigrohr (32) für jede der Röhren (6) des Röhrenbündels.

3. Anlage nach Anspruch 1 oder 2, wobei die Gassteigrohre (32) koaxial zu den Röhren (6) des Röhrenbündels sind.

4. Anlage nach einem der Ansprüche 1 bis 3, wobei die Gassteigrohre (32) einem Boden (30, 30a) in einer unteren Kammer (11) des Abstreifers zugeordnet sind, wobei die untere Kammer (11) mit der Zuführung (12) des Abstreifmediums in Verbindung steht.

5. Anlage nach Anspruch 4, wobei der Boden ein perforierter Boden (30) ist oder einem Rücklauf (37) zugeordnet ist, um eine flüssige Phase der konzentrierten Harnstofflösung (Uc), die aus den Röhren (6) stammt, von dem Boden zu der unteren Kammer (11) unten abzulassen.

6. Verfahren zum Modernisieren eines CO₂-Abstreifers einer Anlage für die Harnstoffsynthese, wobei der Abstreifer, der modernisiert werden soll, einen Einlass einer Harnstofflösung aus einem Synthesereaktor, eine KohlendioxidZufuhr, ein senkrechtes Röhrenbündel von oberflächenbeheizten Röhren umfasst, wobei die Harnstofflösung im Gegenstrom mit Kohlendioxid zugeführt wird und Hülsen oben auf dem Röhrenbündel montiert sind, wobei das Verfahren **dadurch gekennzeichnet ist, dass**
eine Vielzahl von Gassteigrohren (32) vorgesehen sind, die angeordnet sind, um das Kohlendioxid in den jeweiligen Röhren des Bündels zu verteilen, wobei die Gassteigrohre Röhren sind, die kleiner sind als die erwärmten Röhren, wobei jedes Gassteigrohr ein Ende in Verbindung mit der Kohlendioxidzufuhr aufweist und ein zweites Ende, das sich in eine jeweilige Röhre des Röhrenbündels erstreckt.

7. Verfahren nach Anspruch 6, wobei die Gassteigrohre in Verbindung mit einem Boden (30, 30a) vorgesehen sind, wobei der Boden in einer unteren Kammer (11) des Abstreifers angebracht ist, um die konzentrierte Harnstofflösung zu sammeln und in Verbindung mit der Kohlendioxidzuführung ist.

8. Verfahren nach Anspruch 6, wobei jede Röhre des Röhrenbündels direkt mit dem Abstreifmedium mittels eines jeweiligen Gassteigrohres (32) gespeist wird.

## Revendications

1. Installation pour la synthèse de l'urée, comportant au moins un réacteur et un tronçon de revaporisation dans une boucle à haute pression, le tronçon de revaporisation comportant au moins un revaporisateur à enveloppe et à tubes (S_{N}) pour la décomposition du carbamate et la récupération d'ammoniac à partir d'une solution d'urée (U), le revaporisateur comportant une chambre supérieure (2) en communication avec une entrée (1) pour ladite solution d'urée, un faisceau de tubes chauffés (6), lesdits tubes (6) ayant des extrémités supérieures en communication avec la chambre supérieure (2), et des extrémités inférieures (13) en communication avec une alimentation (12) en milieu de revaporisation gazeux, dans lequel, en fonctionnement, un film tombant liquide de ladite solution d'urée et un écoulement gazeux à contre-courant du milieu de revaporisation sont établis à l'intérieur des tubes (6),
**caractérisée en ce que** ladite alimentation (12) du milieu de revaporisation est en communication avec des sections d'extrémité inférieure (34) des tubes (6) au moyen d'une pluralité de conduites montantes de gaz (32) disposées de manière à distribuer le milieu de revaporisation dans des tubes respectifs, chaque conduite montante de gaz étant réalisée avec un tube plus petit que lesdits tubes du faisceau de tubes, ayant une première extrémité en communication avec l'alimentation (12) du milieu de revaporisation, et une seconde extrémité (33) dans un tube respectif (6) du faisceau de tubes, et dans lequel des viroles (5) sont montées sur les extrémités supérieures desdits tubes (6).

2. Installation selon la revendication 1, comportant une conduite montante de gaz (32) pour chacun des tubes (6) dudit faisceau de tubes.

3. Installation selon la revendication 1 ou 2, les conduites montantes de gaz (32) étant coaxiales aux tubes (6) dudit faisceau de tubes.

4. Installation selon l'une quelconque des revendications 1 à 3, les conduites montantes de gaz (32) étant associées à un plateau (30, 30a) dans une chambre inférieure (11) du revaporisateur, ladite chambre inférieure (11) étant en communication avec l'alimentation (12) du milieu de revaporisation.

5. Installation selon la revendication 4, ledit plateau étant un plateau perforé (30) ou étant associé à un trop-plein (37), pour évacuer une phase liquide d'une solution d'urée concentrée (Uc) tombant des tubes (6) en provenance dudit plateau jusqu'à la chambre inférieure (11) située en dessous.

6. Procédé pour moderniser un revaporisateur de CO₂ d'une installation de synthèse d'urée, ledit revaporisateur à moderniser comportant une entrée d'une solution d'urée provenant d'un réacteur de synthèse, une alimentation en dioxyde de carbone, un faisceau de tubes verticaux constitué de tubes chauffés en surface où la solution d'urée est acheminée à contre-courant avec le dioxyde de carbone et des viroles montées sur le dessus du faisceau de tubes, le procédé étant **caractérisé par** la fourniture d'une pluralité de conduites montantes de gaz (32) disposées de manière à répartir le dioxyde de carbone dans des tubes respectifs du faisceau, les conduites montantes de gaz étant des tubes plus petits que les tubes chauffés, chaque conduite montante de gaz ayant une extrémité en communication avec ladite alimentation en dioxyde de carbone, et une seconde extrémité s'étendant dans un tube respectif du faisceau de tubes.

7. Procédé selon la revendication 6, les conduites montantes de gaz étant agencées en association avec un plateau (30, 30a), ledit plateau étant monté dans une chambre inférieure (11) du revaporisateur, pour recueillir la solution d'urée concentrée et en communication avec l'alimentation en dioxyde de carbone.

8. Procédé selon la revendication 6, chaque tube du faisceau de tubes étant directement alimenté avec le milieu de revaporisation au moyen d'une conduite montante de gaz (32) respective.
